# DEMANDE DE BREVET EUROPEEN

(11) **EP 0 860 443 A1**
(43) Date de publication de la demande: **26.08.1998**
(21) Numéro de dépôt: 98400347.5
(22) Date de dépôt: 16.02.1998
(51) Int. Cl.: C07H 17/07

(54) **Procédé de fabrication industrielle de diosmine à partir de l'hesperidine**

(30) Priorité: 21.02.1997 FR 9702055
(71) Demandeur: Innokem, SARL, 94111 Arcueil (FR)
(72) Inventeur: Torregrosa, Jean-Luc, 94230 Cachan (FR); Tembo, Olivier, 95540 Mery Sur Oise (FR); Esprimont Eric, 94110 Arcueil (FR); Favrou, Anita, 94230 Cachan (FR)
(74) Mandataire: Dupuis-Latour, Dominique

(57) **Abrégé**

L'invention concerne un procédé de fabrication industrielle de diosmine à partir de l'hespéridine par réaction avec de l'iode et de la pyridine qui est caractérisé en ce qu'il comprend en combinaison les étapes suivantes dans lesquelles tous les réactifs sont recyclés et consistant à :
a) effectuer une distillation partielle de la pyridine à partir du filtrat résultant de la réaction des composés de départ ;
b) ajouter un hydroxyde de métal alcalin au mélange restant pour obtenir un iodure alcalin en solution dans un mélange eau-pyridine-impuretés ;
c) distiller et traiter séparément l'azéotrope eau-pyridine de l'étape b)
d) recycler en iode l'iodure alcalin résultant des étapes b) et c) en utilisant un acide minéral fort et un oxydant ;
e) séparer par filtration l'iode de l'étape d) et la dissoudre avec la pyridine résultant des étapes a) et f)
f) traiter l'azéotrope résultant eau-pyridine de l'étape c) avec un hydroxyde alcalin pour obtenir après décantation de la pyridine libre et une solution d'hydroxyde alcalin qui est à son tour utilisée dans l'étape b).

## Description

La présente invention concerne un procédé de fabrication industrielle de la diosmine à partir de l'hespéridine avec un recyclage quasiment total des réactifs.

La diosmine est un bioflavonoïde connu à l'état naturel et se trouve par exemple dans l'écorce des agrumes et est utilisée pour son application veinotonique et protectrice du foie. A un niveau industriel l'hémisynthèse de la diosmine est réalisée à partir de l'hespéridine produit naturel.

Les seuls procédés de fabrication connus à ce jour consistent à faire réagir :
- l'hespéridine avec de l'iode en présence de pyridine *(Studies in organic chemistry,* **1981**, vol. 11, 115-119 et le brevet DE 27 40 950) qui fournit la diosmine avec 89 % de rendement ;
- l'hespéridine avec de la soude, de l'iodure de potassium et de l'iode en milieu aqueux *(Studies in organic chemistry,* **1981**, vol. 11, 115-119) qui fournit la diosmine avec un rendement de 50 % ;
- l'hespéridine avec de l'anhydride acétique, de la pyridine et de l'oxyde de sélénium (brevet EP 0 052 086) qui fournit la diosmine avec 79 % de rendement ;
- l'hespéridine avec de l'iode et de la pyridine dans le diméthylformamide (brevet belge 904 614) qui fournit la diosmine avec 75 % de rendement ;
- l'hespéridine avec de l'iode et de la résine de poly-(4-vinylpyridine) dans le diméthylsulfoxyde (brevet belge 904 614) qui fournit la diosmine avec 78 % de rendement ;
- l'hespéridine avec de l'acétate de sodium, de l'iode dans du méthanol (brevet espagnol 537 183) qui fournit la diosmine avec 84 % de rendement et 96 % de pureté ;
- l'hespéridine avec de l'anhydride acétique, de l'acétate de sodium, du brome et de la pyridine (brevet DE 26 02 314) qui fournit la diosmine avec 63 % de rendement contenant 0,09 % de dérivés bromés ;
- l'hespéridine acétylée avec du N-bromosuccinimide ou du bromure de pyridinium et des peroxydes de benzoyle *(J. Org. Chem.,* **1951,** 930) qui fournit la diosmine avec 44 % de rendement.

De plus d'autres procédés de synthèse concernant la déshydrogénation des flavanones en flavones ont été étudiés et décrits dans la littérature comme la combinaison de l'iode avec de l'acide sulfurique dans le diméthylsulfoxyde *(Chem. Ind. (London)* 1979, 315).

Seulement, les procédés décrits présentent de nombreux inconvénients aussi- bien au niveau de leur coût pour les uns que pour leur réalisation industrielle pour les autres :
- pureté ou rendement du produit médiocre ;
- coût des réactifs ou disponibilité à grande échelle problématique ;
- utilisation de l'iode. En effet, la disponibilité de l'iode est critique sur le marché ;
- utilisation de solvants dont la toxicité est importante ou nécessite un équipement coûteux.
- prix du procédé élevé avec l'emploi de réactifs coûteux.

Le procédé offrant l'un des meilleurs résultats pour l'obtention de la diosmine utilise l'iode et la pyridine comme réactifs principaux. La disponibilité de ces deux réactifs est très difficile et en particulier pour l'iode qui ne comporte que deux sources de production : les mines et les saumures. Quant à leur prix de revient, il est très fluctuant suivant la conjoncture économique.

Un procédé industriel utilisant l'iode et la pyridine n'est pas sans risque aussi bien du point de vue de l'approvisionnement en matière première que du coût élevé du retraitement des déchets contenant des iodures et de la pyridine.

Compte tenu de ces problèmes économiques, utiliser un procédé de production basé sur l'utilisation de l'iode et de la pyridine nécessite un moyen de recyclage fiable de ces réactifs et est nécessaire pour assurer une autonomie de production et baisser le prix de revient de la production.

A la suite d'études approfondies en vue de mettre au point un procédé échappant aux inconvénients susmentionnés, on a trouvé un système de recyclage presque quantitatif de la majorité des réactifs et en particulier de l'iode et de la pyridine et permettant une diminution importante du coût de fabrication ainsi qu'une solution aux problèmes d'approvisionnement.

La présente invention a donc pour objet un procédé de fabrication industrielle de diosmine à partir de l'hespéridine par réaction avec de l'iode et de la pyridine, lequel procédé est caractérisé en ce qu'il comprend en combinaison les étapes suivantes dans lesquelles tous les réactifs sont recyclés et consistant à :
a) effectuer une distillation partielle de la pyridine à partir du filtrat résultant de la réaction des composés de départ ;
b) ajouter un hydroxyde de métal alcalin au mélange restant pour obtenir un iodure alcalin en solution dans un mélange eau-pyridine-impuretés ;
c) distiller et traiter séparément l'azéotrope eau-pyridine de l'étape b) ;
d) recycler en iode l'iodure alcalin résultant des étapes b) et c) en utilisant un acide minéral fort et un oxydant ;
e) séparer par filtration l'iode de l'étape d) et la dissoudre avec la pyridine résultant des étapes a) et f) ;
f) traiter l'azéotrope résultant eau-pyridine de l'étape c) avec un hydroxyde alcalin pour obtenir après décantation de la pyridine libre et une solution d'hydroxyde alcalin qui est à son tour utilisée dans l'étape b).

L'invention est également relative aux caractéristiques ci-après prises isolément ou en combinaison :
- l'hydroxyde de métal alcalin est choisi parmi l'hydroxyde de sodium et l'hydroxyde de potassium ;
- l'hydroxyde de métal alcalin est utilisé dans l'étape b) à raison d'une concentration comprise dans la gamme de 20 à 30 % ;
- l'acide minéral fort est choisi parmi l'acide sulfurique, l'acide chlorhydrique, l'acide nitrique et l'acide phosphorique ;
- l'oxydant est choisi parmi H₂O₂, MnO₂, HClO₄, KMnO₄ et NaIO₃;
- l'acide minéral fort est utilisé dans le recyclage de l'iodure alcalin jusqu'à l'obtention d'un pH compris dans la gamme de 0,5 à 2 ;
- l'oxydant utilisé dans le recyclage de l'iodure alcalin en iode est de l'eau oxygénée ;
- la concentration d'hydroxyde de métal alcalin utilisée dans le recyclage de l'azéotrope eau-pyridine de l'étape f) est comprise dans la gamme de 40 à 60 % ;
- la durée de la réaction se situe dans la gamme de 4 heures à 6 heures ;
- la température de la réaction est comprise dans la gamme de 90°C à 118°C ;
- le taux de recyclage d'iode est compris dans la gamme de 70 à 95 % tandis que les taux de recyclage de la pyridine et de l'eau sont compris dans la gamme de 95 à 99 % ;
- la diosmine obtenue est, en outre, purifiée par un traitement avec une base organique ou minérale.

Selon la présente invention, la synthèse de la diosmine à partir de l'hespéridine s'effectue en présence de pyridine et d'iode. En fin de réaction, la diosmine récupérée nécessite une recristallisation en présence d'une base minérale ou organique et d'un acide. En même temps que l'obtention de la diosmine, produit solide obtenu après filtration, on obtient dans le filtrat une solution d'iodure de pyridinium et d'impuretés de la diosmine dans un mélange eau-pyridine.

A partir de ce mélange on a trouvé un système de recyclage avec de bons taux de la pyridine, de l'iode et de l'eau utilisant une quantité limitée de réactifs : un hydroxyde alcalin (de la soude préférentiellement), un acide (de l'acide sulfurique, préférentiellement) et un oxydant de préférence, de l'eau oxygénée à 30 %.

Tout d'abord une distillation partielle (60 %) de la pyridine est opérée à partir de ce filtrat. Puis sur le mélange restant un hydroxyde alcalin (hydroxyde de sodium) en solution dans l'eau est ajouté pour donner un iodure alcalin (iodure de sodium) en solution dans un mélange eau-pyridine avec les impuretés.

L'azéotrope eau-pyridine est distillé et est traité séparément. Il ne reste donc à présent dans la solution que l'iodure alcalin, les impuretés et l'eau. L'iodure alcalin est recyclé en iode en utilisant de l'acide sulfurique et de l'eau oxygénée. Selon l'invention, une étude a été réalisée sur le recyclage de l'iodure de pyridinium pur, et on a observé un rendement de 92 %.

L'iode est filtré à partir de la solution aqueuse et est dissous avec de la pyridine (distillée auparavant) et le mélange réactionnel pyridine-iode est prêt à l'emploi pour le cycle suivant.

L'azéotrope eau-pyridine est traité avec un hydroxyde alcalin et on obtient après décantation la pyridine libre et une solution d'hydroxyde alcalin qui peut à son tour être réutilisée pour le passage de l'iodure de pyridinium en iodure alcalin. Préférentiellement l'hydroxyde alcalin utilisé est l'hydroxyde de sodium.

De plus à partir de toutes les solutions aqueuses une distillation de l'eau est pratiquée et est réinjectée dans le procédé.

La présente invention fournit donc un procédé de fabrication de la diosmine d'un prix de revient peu onéreux dans lequel on prévoit :
- la synthèse de la diosmine à partir de l'hespéridine en utilisant de l'iode et de la pyridine ;
- le recyclage des réactifs employés c'est à dire l'iode, la pyridine et l'eau ;
- l'utilisation de peu de réactifs supplémentaires pour le recyclage de l'iode et de la pyridine : un oxydant, un acide et une base ;
- la recristallisation de la diosmine avec une base organique ou minérale (préférentiellement de la morpholine ou de la soude) et un acide (préférentiellement de l'acide acétique).

On indique ci-après les conditions de mise en oeuvre et les concentrations des réactifs utilisés :

Le dosage de l'iode utilisé dans la réaction selon l'invention est compris entre 1,3 et 2 équivalents mais il est préférentiellement utilisé 1,6 équivalent molaire.

Concernant l'hydroxyde alcalin, il est préférable d'utiliser l'hydroxyde de sodium ou l'hydroxyde de potassium.

Le dosage de l'hydroxyde alcalin utilisé dans le recyclage de l'iodure de pyridinium selon l'invention est compris entre 20 % et 30 % mais il est préférentiellement utilisé une concentration de 27 %.

Le dosage de l'acide sulfurique utilisé dans le recyclage de l'iodure alcalin en iode selon l'invention est compris jusqu'à l'obtention d'un pH compris entre 0,5 et 2 mais préférentiellement inférieur à 1.

Le dosage de l'eau oxygénée dans le recyclage de l'iodure alcalin en iode selon l'invention est indexé sur l'évolution du pH. Il est ajouté de l'eau oxygénée jusqu'à la stabilité du pH. L'eau oxygénée utilisée est à 30 % garantissant ainsi une meilleure sécurité.

Le dosage de la soude utilisée dans le recyclage de l'azéotrope eau-pyridine selon l'invention est compris entre 20 et 35 % mais la plage 27-31 est préférée. Son volume est généralement compris entre 40 et 60 % du volume total de l'azéotrope mais il est préférentiellement utilisé en quatre fois 50 % du volume total de l'azéotrope.

Concernant le temps de réaction selon l'invention, il a été trouvé que le temps de réaction était compris entre 4 et 6 heures mais est préférentiellement de 5 heures.

La température de réaction selon l'invention est comprise entre 90 et 118°C mais est préférentiellement entre 110 et 115°C.

Ce procédé permet d'obtenir la diosmine avec un rendement de 75 % et une qualité pharmacopée à un coût de revient très inférieur à celui utilisant les procédés de l'art antérieur. Le taux de recyclage de l'iode est compris entre 70 et 95 %, celui de la pyridine et de l'eau est compris entre 95 et 99 %.

Le procédé selon l'invention permet donc de synthétiser la diosmine à partir de l'hespéridine en présence de pyridine et d'iode et avec une étape de recristallisation de la diosmine avec de la soude et de l'acide acétique.

Le système de recyclage presque total de l'iode, de la pyridine et de l'eau confère un très grand intérêt industriel à ce procédé en lui donnant une grande autonomie de production et en abaissant le prix de revient de la production de la diosmine.

L'invention sera mieux comprise à la lecture de l'exemple d'exécution qui suit.

### Exemple

Dans un tricol de 250 ml, on place 20 g d'hespéridine, 13,30 g d'iode et 100 ml de pyridine. La solution noire obtenue est portée à reflux pendant 5 heures. Au bout de 1 heure 30 minutes de reflux environ, la diosmine commence à précipiter dans le milieu. Au bout des 5 heures de reflux, le milieu réactionnel est refroidi à 5°C et filtré sur fritté de porosité 4. On obtient un solide humide marron et un filtrat noir (volume 72 ml). Le solide marron est lavé avec 3 fois 35 ml d'eau pour donner un solide beige (diosmine brute) et un filtrat aqueux (volume 99 ml) marron.

### Recyclage de la pyridine

Le filtrat noir est distillé pour récupérer la pyridine : on récupère 52 ml de pyridine.

Au résidu restant dans le bouilleur, est additionné le filtrat marron et 5,25 g de soude en pastilles. On distille alors l'azéotrope eau-pyridine (volume de 70 ml). L'azéotrope eau-pyridine est alors extrait 4 fois avec de la soude à 50 % (volume de 20,7 ml). Il est récupéré 41 ml de phase organique et 49,7 ml de phase aqueuse sodée de concentration 27 %.

L'eau de la phase organique est éliminée par distillation d'un peu d'azéotrope eau-pyridine, il est récupéré ainsi 40 ml de pyridine.

Les fluides de pyridine récupérée peuvent être utilisés dans l'étape suivante de synthèse.

### Recyclage de l'iode

Le résidu restant dans le bouilleur après distillation de l'azéotrope eau-pyridine, est acidifié par ajout de 9,5 ml d'acide sulfurique à 50 % en volume. Le pH obtenu est inférieur à 1. Il se forme un précipité brun qui est filtré et considéré en tant que déchets. Le filtrat obtenu est oxydé par addition de 6,7 ml d'eau oxygénée à 30 %. L'addition dure 45 minutes, à une température inférieure à 10°C. L'agitation est maintenue 30 minutes après la fin de l'addition. Le milieu prend instantanément une couleur rouge, puis il se forme un précipité noir qui est filtré sur fritté de porosité 4.

On obtient un filtrat rouge/marron qui est récupéré pour l'évaporation de l'eau (volume de 44,7 ml).

Le précipité noir obtenu (iode recyclé) est remis en solution sur le fritté dans les fluides de pyridine récupérée.

### Purification de la diosmine brute

La diosmine brute est dissoute dans 15,3 ml de la phase aqueuse sodée de concentration 27 % (solution de soude) à 50°C. Après dissolution, la température est montée à 70°C et 14 ml d'acide acétique à 99 % sont additionnés. La température est maintenue à 70°C pendant une heure. La diosmine a reprécipité. Le milieu réactionnel est refroidi à 5°C, puis filtré sur fritté de porosité 4. On obtient un solide beige qui est lavé avec 100 ml d'eau en 3 fois. Cette eau est récupérée pour l'évaporation. Après un passage à l'étuve pendant 24 heures à 60°C, on obtient la diosmine avec un rendement de 75 % et une pureté supérieure à 90 %.

Les cycles suivants sont exécutés de la même manière à partir des solutions recyclées auparavant avec un apport supplémentaire de 25 % en iode et entre 2 et 4 % en pyridine.

La présente invention fournit donc un procédé de fabrication industrielle de la diosmine à partir de l'hespéridine avec un recyclage presque total des réactifs employés conduisant à une baisse considérable du prix de revient.

## Revendications

1. Procédé de fabrication industrielle de diosmine à partir de l'hespéridine par réaction avec de l'iode et de la pyridine, caractérisé en ce qu'il comprend en combinaison les étapes suivantes dans lesquelles tous les réactifs sont recyclés et consistant à :
a) effectuer une distillation partielle de la pyridine à partir du filtrat résultant de la réaction des composés de départ ;
b) ajouter un hydroxyde de métal alcalin au mélange restant pour obtenir un iodure alcalin en solution dans un mélange eau-pyridine-impuretés ;
c) distiller et traiter séparément l'azéotrope eau-pyridine de l'étape b) ;
d) recycler en iode l'iodure alcalin résultant des étapes b) et c) en utilisant un acide minéral fort et un oxydant ;
e) séparer par filtration l'iode de l'étape d) et la dissoudre avec la pyridine résultant des étapes a) et f) ;
f) traiter l'azéotrope résultant eau-pyridine de l'étape c) avec un hydroxyde alcalin pour obtenir après décantation de la pyridine libre et une solution d'hydroxyde alcalin qui est à son tour utilisée dans l'étape b).

2. Procédé selon la revendication 1, caractérisé en ce que l'hydroxyde de métal alcalin est choisi parmi l'hydroxyde de sodium et l'hydroxyde de potassium.

3. Procédé selon l'une des revendications 1 ou 2, caractérisé en ce que l'hydroxyde de métal alcalin est utilisé dans l'étape b) à raison d'une concentration comprise dans la gamme de 20 à 30 %.

4. Procédé selon la revendication 1, caractérisé en ce que l'acide minéral fort est choisi parmi l'acide sulfurique, l'acide chlorhydrique, l'acide nitrique et l'acide phosphorique.

5. Procédé selon la revendication 1, caractérisé en ce que l'oxydant est choisi parmi H₂O₂, MnO₂, HClO₄ , KMnO₄ et NaIO₃.

6. Procédé selon l'une quelconque des revendications 1 à 4, caractérisé en ce que l'acide minéral fort est utilisé dans le recyclage de l'iodure alcalin jusqu'à l'obtention d'un pH compris dans la gamme de 0,5 à 2.

7. Procédé selon l'une quelconque des revendications 1 à 6, caractérisé en ce que l'oxydant utilisé dans le recyclage de l'iodure alcalin en iode est de l'eau oxygénée.

8. Procédé selon la revendication 1, caractérisé en ce que la concentration d'hydroxyde de métal alcalin utilisée dans le recyclage de l'azéotrope eau-pyridine de l'étape f) est comprise dans la gamme de 40 à 60 %.

9. Procédé selon l'une quelconque des revendications 1 à 8, caractérisé en ce que la durée de la réaction se situe dans la gamme de 4 heures à 6 heures.

10. Procédé selon l'une quelconque des revendications 1 à 9, caractérisé en ce que la température de la réaction est comprise dans la gamme de 90°C à 118°C.

11. Procédé selon l'une quelconque des revendications 1 à 10, caractérisé en ce que le taux de recyclage de l'iode est compris dans la gamme de 70 à 95 % tandis que les taux de recyclage de la pyridine et de l'eau sont compris dans la gamme de 95 à 99 %.

12. Procédé de fabrication industrielle de diosmine dans lequel la diosmine obtenue à partir du procédé selon l'une quelconque des revendications précédentes est, en outre, purifiée par un traitement avec une base organique ou minérale.
